Europäisches Patentamt

European Patent Office

Office européen des brevets

(19)

(11) Numéro de publication: **0 478 450 B1**

(12) **FASCICULE DE BREVET EUROPEEN**

(45) Date de publication de fascicule du brevet: **14.12.94**

(51) Int. Cl.5: **C12Q  1/68**, C07K 3/00,
G01N 33/68

(21) Numéro de dépôt: **91402550.7**

(22) Date de dépôt: **25.09.91**

(54) **Procédé de séquencage rapide de séquences biologiques linéaires et ordonnées.**

(30) Priorité: **28.09.90 FR 9011977**

(43) Date de publication de la demande:
**01.04.92 Bulletin  92/14**

(45) Mention de la délivrance du brevet:
**14.12.94 Bulletin  94/50**

(84) Etats contractants désignés:
**AT BE CH DE DK ES FR GB GR IT LI LU NL SE**

(56) Documents cités:
**WO-A-89/03432
WO-A-90/04652**

**PROCEEDINGS OF THE NATL. ACADEMY OF
SCIENCES USA, vol. 76,no. 5, mai 1979; M.O.
DAYHOFF et al., pp. 2170-2174&NUM;**

**JAPANESE PATENTS GAZETTE, semaine
8822, 13 juillet 1988, unexamined, section
Ch/Chemical, Derwent Publications Ltd.,
Londres (GB); p. 14, no. 88-151045/22&NUM;**

(73) Titulaire: **BERTIN & CIE
Zone Industrielle
Boîte postale 3
F-78373 Plaisir Cédex (FR)**

(72) Inventeur: **Ruggiu, Gilles
113 avenue des Bleuets
F-91400 Orsay (FR)**
Inventeur: **Ginot, Frédéric
17 rue Neuve Notre Dame
F-78000 Versailles (FR)**

(74) Mandataire: **Orès, Bernard et al
Cabinet ORES
6, Avenue de Messine
F-75008 Paris (FR)**

EP 0 478 450 B1

## Description

La présente invention est relative à un procédé de séquençage rapide de séquences biologiques linéaires et/ou préalablement linéarisées et ordonnées, notamment des protéines et des acides nucléiques.

Les méthodes actuelles de détermination tant des protéines que des acides nucléiques sont lourdes à mettre en oeuvre.

Pour la détermination des protéines, on peut citer notamment la méthode d'Edman.

Pour la détermination de la séquence d'un acide nucléique, il existe à l'heure actuelle, schématiquement deux grands types de méthodes : les méthodes chimiques et les méthodes enzymatiques.

La méthode chimique, représentée essentiellement par la méthode de Maxam et Gilbert, est basée sur l'obtention de fragments dont la taille permet de définir la position d'une des quatre bases. Avant toute détermination, l'ADN est purifié sous forme simple-brin, puis marqué à l'une de ses extrémités par le $^{32}$P. Il est alors séparé en quatre sous-ensembles qui subissent chacun un traitement chimique différent, qui altère de façon absolument spécifique un type de base (modification de la base, élimination de la base modifiée et coupure du brin au niveau du résidu sucré) et entraîne le clivage de ladite séquence en deux fragments. Les paramètres chimiques sont choisis de façon à ce que chaque fragment ait en moyenne une seule base modifiée, puis éliminée. Après clivage, chaque sous-ensemble contient donc une population de fragments marqués de taille variable, ayant tous une même extrémité et se terminant par un même type de base dans la séquence.

Après séparation électrophorétique et révélation par autoradiographie, la longueur du segment observé est égale à la distance entre le point de coupure et le point de marquage. Comme la résolution des gels de polyacrylamide utilisés est d'une base, l'enchaînement des fragments sur l'autoradiogramme correspond à l'enchaînement des bases dans le fragment d'ADN.

La lecture est directe et une séquence de 100 à 400 bases peut être lue sur un seul gel.

Cette méthode est plutôt adaptée aux fragments de l'ordre de 500 pb, présents en assez grande quantité et pour lesquels les structures secondaires sont importantes.

La méthode enzymatique, essentiellement représentée par la méthode de Sanger, consiste à faire synthétiser par une enzyme appropriée (polymérase ou transcriptase réverse), le brin complémentaire au brin que l'on désire séquencer et qui doit être intégré dans un vecteur simple ou double-brin susceptible de se répliquer.

La synthèse du second brin est effectuée en présence des 4 désoxyribonucléotides (dATP, dCTP, dTTP, dGTP), dont au moins un doit être marqué radioactivement, et d'une amorce qui va s'hybrider à une région juste en amont du fragment à séquencer.

Le principe de cette technique consiste en l'addition d'un didésoxynucléotide spécifique différent (ddATP, ddCTP, ddTTP ou ddGTP) dans quatre tubes de réaction. Ces ddNTPs peuvent être incorporés dans la chaîne en élongation, mais ne possédant pas de groupement hydroxyle en 3', leur incorporation arrête l'élongation du brin. Chaque tube réactionnel contient donc, en fin de réaction, une population de fragments de taille variable ayant tous la même extrémité 5′ et se terminant par le même didésoxynucléotide (ddNTP). La taille des fragments synthétisés, donc la taille des fragments détectables par autoradiographie après électrophorèse est égale à la distance entre le début de l'amorce et la base où s'est arrêtée la réplication.

Il est possible, par cette technique, de déterminer des séquences de 300 à 800 bases suivant la longueur et la qualité des gels de polyacrylamide qui servent à la séparation.

D'autres méthodes ont été proposées pour permettre de réduire les étapes de sous-clonage (séquençage du fragment double-brin, utilisation de la PCR, séquençage en shot gun, par exemple).

La méthode en shot gun, en particulier, consiste à cloner des fragments du gène à séquencer au hasard dans M13 sans avoir aucune information sur leur organisation dans le génome.

Les fragments sont ensuite séquencés et leur agencement se fait par recoupement à l'aide d'un ordinateur ; toutefois, cette méthode nécessite le séquençage direct d'au moins 20 % des séquences que l'on n'a pas pu replacer par recoupage.

Etant donné la difficulté d'obtention des séquences manquantes, des adaptations plus performantes ont été mises au point ; on peut citer brièvement la méthode de séquençage par clonage et délétions enzymatiques et la méthode de séquençage par délétions provoquées par un transposon.

Toutefois, toutes ces méthodes sont lourdes à mettre en oeuvre.

La Demande Internationale PCT WO 89/03432 décrit, pour sa part, une méthode de séquençage rapide d'ADN et d'ARN, dans laquelle un fragment simple brin d'ADN ou d'ARN éventuellement fixé sur un support solide est mis dans un liquide en flux et est clivé par une exonucléase, à partir de l'une de ses extrémités, de manière à former une suite de bases dans l'échantillon en flux. Les bases sont ensuite détectées lors de leur passage séquentiel à travers un détecteur, pour reconstruire la séquence en bases dudit fragment d'ADN ou d'ARN à déterminer. Dans un mode de réalisation particulier, le brin complémentaire à la séquence à déterminer, est synthétisé en présence de nucléotides modifiés et possédant chacun une caractéristique spécifique (fluorescence différente). Le fragment synthétisé est ensuite mis sur un support solide dans l'échantillon liquide en flux et les différents nucléotides identifiables sont clivés séquentiellement et détectés.

Cependant, dans un tel procédé, l'ensemble des bases ne peut pas en fait être marqué, notamment pour des raisons d'encombrement stérique.

L'invention a pour objet un procédé de séquençage de séquences biologiques linéaires ou linéarisées et ordonnées qui réponde mieux aux nécessités de la pratique que les procédés de l'Art antérieur, notamment en permettant d'obtenir beaucoup plus rapidement que dans les procédés antérieurement décrits la séquence complète, à partir de fragments marqués partiellement.

L'invention propose à cet effet un procédé de séquençage des éléments constituant une séquence biologique linéaire ou linéarisée et ordonnée, le procédé consistant notamment à extraire, à purifier et éventuellement à fragmenter et/ou à amplifier une séquence biologique pour obtenir une pluralité de séquences identiques, à associer aux éléments de ces séquences des marqueurs spécifiques appropriés, puis à identifier séquentiellement et dénombrer les marqueurs associés auxdits éléments pour en déduire une séquence complète d'éléments, caractérisé en ce qu'il consiste :

- à marquer lesdites séquences par l'ensemble des marqueurs spécifiques précités, l'efficacité du marquage étant inférieure à 100 %,
- à détecter et identifier séquentiellement les marqueurs associés aux éléments dans lesdites séquences, pour obtenir des séquences lacunaires ou incomplètes d'éléments marqués, qui diffèrent les unes des autres par les éléments marqués, qui comprennent chacune un nombre d'éléments marqués inférieur au nombre N d'éléments constituant ladite séquence complète, et dans chacune desquelles les intervalles entre éléments marqués ont des longueurs inconnues, et
- à reconstituer la suite ordonnée d'éléments constituant la séquence complète, en calculant à partir desdites séquences lacunaires les probabilités de présence des éléments dans la séquence complète, en partant de valeurs initiales prédéterminées de ces probabilités et en tenant compte des éléments marqués présents dans une première séquence lacunaire, puis en modifiant les probabilités ainsi obtenues en tenant compte des éléments marqués présents dans une deuxième séquence lacunaire, et ainsi de suite, jusqu'à arriver à des probabilités égales à 1 ou voisines de 1 pour chaque élément de la séquence complète, les calculs précités étant effectués en procédant élément par élément et emplacement par emplacement de la séquence complète, en calculant la probabilité de présence de chaque élément possible pour un emplacement donné, puis la probabilité de présence de chacun des éléments possibles pour l'emplacement suivant dans la séquence complète, et ainsi de suite, la probabilité de présence d'un élément Bj en un emplacement i dans la séquence complète étant égale à :

$$P_p^i (Bj) = \frac{P_{p-1}^i(Bj) \cdot P(S_p/Bj)}{\sum\limits_{j=1}^{4} P_{p-1}^i(Bj) \cdot P(Sp/Bj)} \, ,$$

$P_p^i$ (Bj) étant la probabilité de la présence de l'élément Bj à l'emplacement i de la séquence complète après considération de p séquences lacunaires,

$$P_{p-1}^i \ (Bj)$$

étant la probabilité de la présence de l'élément Bj à l'emplacement i de la séquence complète après considération de (p-1) séquences lacunaires,

$P(S_p/Bj)$ étant la probabilité de la séquence lacunaire $S_p$, connaissant l'élément Bj à l'emplacement i dans la séquence complète.

On entend par séquence biologique linéaire ou linéarisée, au sens de la présente invention, aussi bien les protéines, dont les éléments sont les acides aminés naturels, que les acides nucléiques (ADN et ARN), dont les éléments sont les bases ou nucléotides : adénine, cytosine, guanine, thymidine, symbolisées par les lettres A, C, G et T pour l'ADN et A, C, G et U (uracile) pour l'ARN. Pour des raisons de simplicité, dans la suite, l'ARN sera symbolisé par les mêmes lettres que l'ADN.

Cette façon de faire simplifie les calculs et réduit largement le temps de traitement sur un ordinateur ou un micro-ordinateur.

Selon une caractéristique avantageuse de l'invention, le procédé consiste, préalablement, à déterminer le nombre N d'éléments de la séquence complète.

Selon une autre caractéristique avantageuse de l'invention, le procédé consiste préalablement à déterminer, pour chaque type d'élément, le nombre total d'éléments de ce type dans la séquence complète, et ce, indépendamment de leur position dans la séquence.

Selon une disposition avantageuse de l'invention, le nombre d'éléments de chaque type dans la séquence complète est déterminé par voie chimique.

On peut ainsi initialiser le calcul des probabilités à partir du nombre d'éléments de chaque type dans la séquence complète, la valeur initiale de la probabilité d'un élément Bj en un emplacement étant égale à nj/N, nj étant le nombre total d'élément Bj dans la séquence complète et N le nombre total d'éléments dans cette séquence.

Pour que le calcul puisse s'effectuer assez rapidement, le nombre N est choisi par exemple compris entre 10 et 30 environ.

Dans le cas d'une séquence biologique qui comprend un grand nombre d'éléments, on considère des séquences complètes de N éléments qui se recouvrent mutuellement à leurs extrémités, N étant compris entre 10 et 30 environ et on détermine, de la façon précitée, les suites d'éléments constituant ces séquences successives, ce qui permet de reconstituer la séquence biologique complète considérée.

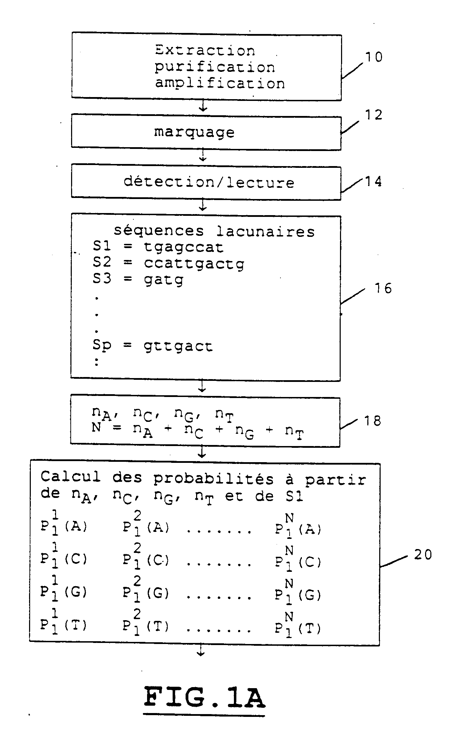

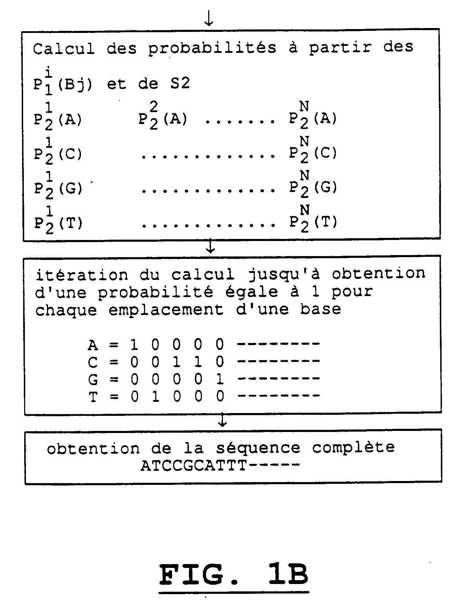

L'invention sera mieux comprise et d'autres caractéristiques, détails et avantages de celle-ci apparaîtront plus clairement à la lecture de la description qui suit, faite à titre d'exemple en référence aux dessins annexés, dans lesquels les figures 1A et 1B représentent les principales étapes du procédé selon l'invention, appliqué au séquençage d'un acide nucléique.

Une première étape du procédé, désignée par la référence 10 en figure 1A, consiste de façon classique à extraire et à purifier un fragment d'acide nucléique, et éventuellement à l'amplifier lorsqu'il est présent en très petites quantités, par exemple par la méthode PCR.

La seconde étape du procédé, désignée par la référence 12, consiste à associer aux bases des fragments d'acide nucléique des marqueurs spécifiques qui, pour des raisons d'encombrement stérique, ne pourront pas être associés à toutes les bases de ces fragments.

On peut, de façon connue, procéder à des copies du fragment d'ADN par une polymérase appropriée, en présence des nucléotides A, C, G et T et d'analogues des nucléotides marqués spécifiquement par des fluorochromes différents appelés ci-après a, c, g et t : a marque spécifiquement l'adénine, c marque spécifiquement la cytosine, g marque spécifiquement la guanine et t marque spécifiquement la thymidine.

On obtient ainsi un ensemble de fragments d'ADN qui sont marqués partiellement et différemment, les bases marquées n'étant pas les mêmes dans les différents fragments, les nombres de bases marquées différant également d'un élément à l'autre.

L'étape suivante du procédé, désignée par la référence 14, consiste à détecter ou à lire les bases marquées. On peut pour cela étaler les différents fragments d'ADN sur une lame de microscope, et détecter les marqueurs associés à des bases par exemple au moyen d'un microscope à effet tunnel ou par holographie conoscopique.

On obtient ainsi, comme représenté en 16, un ensemble de séquences lacunaires S1, S2, S3, ..., Sp, ..., de bases marquées, dans lequel une séquence lacunaire va différer d'une autre par le nombre de bases marquées, les emplacements des bases marquées, et les intervalles entre bases marquées.

Selon l'invention, la connaissance de cet ensemble de séquences lacunaires ou incomplètes de bases marquées permet de remonter à la séquence complète de bases constituant le fragment d'acide nucléique étudié.

On va, pour cela, calculer la probabilité de présence de chaque base possible A, C, G ou T en chaque emplacement i d'une base dans la séquence complète à partir des informations contenues dans les séquences lacunaires précitées, qui sont des suites incomplètes, mais ordonnées de bases marquées.

Théoriquement, la formule de BAYES permet de calculer la probabilité d'une combinaison quelconque Ck de N éléments, connaissant un ensemble S de suites partielles d'éléments tirées d'une combinaison particulière :

4

$$P(Ck/S) = \frac{P(Ck).P(S/Ck)}{\sum_k P(Ck).P(S/Ck)}$$

avec

: $P(Ck/S)$ =     probabilité d'une combinaison quelconque Ck connaissant S

: $P(Ck)$ =     probabilité d'une combinaison Ck

: $P(S/Ck)$ =     probabilité de S connaissant Ck

L'application de cette formule dans le cas présent est pratiquement impossible, en raison du volume de calculs à effectuer : si l'on considère en effet une séquence de N bases, le nombre de combinaisons Ck est égal à $4^N$, c'est-à-dire qu'il est supérieur à $10^6$ lorsque N est supérieur à 10.

L'invention prévoit donc de simplifier ces calculs, afin de réduire largement leur durée sur un système de traitement de l'information, en procédant par exemple de la façon suivante :

- on effectue les calculs en considérant chaque séquence lacunaire S1, S2, ..., Sp, ... l'une après l'autre, au lieu de considérer l'ensemble S de ces séquences lacunaires,
- on calcule, non pas la probabilité d'une séquence possible de N bases, mais la probabilité de chaque base possible en chaque emplacement de base du fragment d'acide nucléique considéré.

Ces deux simplifications permettent d'arriver à la formule suivante :

$$P^i_p(Bj) = \frac{P^i_{p-1}(Bj).P(Sp/Bj)}{\sum_{j=1}^{4} P^i_{p-1}(Bj).P(Sp/Bj)}$$

avec

$P^i_p(Bj)$ = probabilité de la base Bj à l'emplacement i de la séquence complète après considération des p premières séquences lacunaires.

$$P^i_{p-1}(Bj)$$

= probabilité de la base Bj à l'emplacement i de la séquence complète après considération des (p-1) premières séquences lacunaires.

$P(Sp/Bj)$ = probabilité de la séquence lacunaire Sp connaissant la base Bj à l'emplacement i de la séquence complète.

$Bj = A, C, G$ ou $T$.

Un tel calcul est réalisable sans difficulté sur un micro-ordinateur pour des valeurs de N comprises entre 10 et 30 environ, N étant le nombre de bases de la séquence complète recherchée.

On peut initialiser ce calcul en considérant que toutes les bases ont des probabilités égales (1/4) de se trouver en un emplacement donné de la séquence.

Il est cependant plus avantageux d'initialiser le calcul en partant de probabilités qui sont plus proches de la réalité. On peut pour cela déterminer, par exemple par un procédé chimique, les nombres $n_A$, $n_C$, $n_G$, $n_T$ et le nombre N total de bases A, C, G, T dans la séquence étudiée.

Cette étape est désignée par la référence 18 en figure 1A.

L'étape suivante du procédé, réalisée sur un micro-ordinateur par exemple, consiste à appliquer la formule précitée.

Comme indiqué en 20 en figure 1A, on calcule donc les probabilités de présence de chaque base possible en chaque emplacement de la séquence complète, à partir des nombres de bases de chaque type $n_A$, $n_C$, $n_G$, $n_T$ et de la première séquence lacunaire S1. Les résultats de ce calcul peuvent être présentés sous forme d'un tableau ou d'une matrice de valeurs, dans lequel la première ligne indique les probabilités de présence de la base A aux divers emplacements de bases dans la séquence complète, la deuxième ligne indique les probabilités de présence de la base C aux divers emplacements de la séquence complète, la troisième ligne indique les probabilités de présence de la base G aux divers emplacements de cette séquence complète, et la quatrième ligne indique les probabilités de présence de la base T aux divers emplacements de la séquence complète.

Par itérations successives et en considérant successivement les séquences lacunaires S2, ..., Sp, ... on arrive à un tableau qui va être composé uniquement de 1 et de 0 (ou de chiffres très voisins de 1 et de chiffres très voisins de 0), les 1 identifiant les bases présentes aux divers emplacements à la séquence complète, les 0 confirmant que d'autres bases ne se trouvent pas à ces mêmes emplacements.

Les séquences lacunaires précitées peuvent bien entendu être considérées dans un ordre quelconque. Il est cependant plus avantageux de les considérer par ordre décroissant de nombre de bases marquées, afin de bénéficier d'un maximum d'informations dès le début des calculs.

Il est intéressant également, après avoir effectué les calculs correspondant à un certain nombre de séquences lacunaires, de continuer ces calculs en reprenant les séquences lacunaires les plus riches en informations.

Il en résulte une réduction du temps de calcul qui peut être relativement importante.

Le nombre de séquences lacunaires à considérer pour la reconstitution d'une séquence complète, varie avec l'efficacité du marquage des bases de cette séquence complète. Pour une efficacité de marquage de l'ordre de 30 %, il faudra considérer une vingtaine de séquences lacunaires quelconques, ce nombre pouvant être réduit si l'on selectionne les séquences lacunaires les plus riches en informations. La durée des calculs sur un micro-ordinateur de type classique peut varier de quelques minutes à quelques dizaines de minutes, selon les cas.

Les séquences complètes étudiées peuvent être de longueur quelconque. Si le procédé selon l'invention est mis en oeuvre à l'aide d'un micro-ordinateur, il sera avantageux de limiter ce nombre à 30 environ. Dans le cas où l'on étudie des séquences de bases relativement longues, il suffit de décomposer ces séquences complètes en séquences successives de 20 à 30 bases environ qui se chevauchent à leurs extrémités, et de déterminer la constitution de ces séquences successives.

De façon générale, l'invention est applicable chaque fois que l'on doit reconstituer une séquence biologique linéaire et ordonnée à partir d'informations ordonnées, mais incomplètes. Elle s'applique donc aussi bien au séquençage des acides nucléiques qu'à celui des protéines.

## Revendications

1. Procédé de séquençage des éléments constituant une séquence biologique linéaire ou linéarisée et ordonnée, telle par exemple qu'un fragment d'ADN, ce procédé consistant notamment à extraire, à purifier et éventuellement à fragmenter et/ou amplifier ladite séquence biologique pour obtenir une pluralité de séquences identiques, à associer aux éléments constituant ces séquences des marqueurs spécifiques appropriés, puis à identifier séquentiellement et dénombrer les marqueurs associés auxdits éléments pour en déduire une séquence complète d'éléments, caractérisé en ce qu'il consiste :
   - à marquer lesdites séquences par l'ensemble des marqueurs spécifiques précités, l'efficacité de ce marquage étant inférieure à 100 %,
   - à détecter et identifier séquentiellement les marqueurs associés aux éléments desdites séquences, pour obtenir des séquences lacunaires ou incomplètes d'éléments marqués, qui diffèrent les unes des autres par les éléments marqués, qui comprennent chacune un nombre d'éléments marqués inférieur au nombre N d'éléments constituant ladite séquence biologique, et dans chacune desquelles les intervalles entre éléments marqués ont des longueurs inconnues, et
   - à reconstituer la suite ordonnée d'éléments constituant la séquence complète, en calculant à partir desdites séquences lacunaires les probabilités de présence des éléments dans la séquence complète, en partant de valeurs initiales prédéterminées de ces probabilités et en tenant compte des éléments marqués présents dans une première séquence lacunaire, puis en modifiant les probabilités ainsi obtenues en tenant compte des éléments marqués présents dans une deuxième séquence lacunaire, et ainsi de suite, jusqu'à arriver à des probabilités égales à 1 ou voisines de 1 pour chaque élément de la séquence complète, les calculs précités étant effectués en procédant élément par élément et emplacement par emplacement de la séquence complète, en calculant la probabilité de présence de chaque élément possible pour un emplacement donné, puis la probabilité de présence de chacun des éléments possibles pour l'emplacement suivant dans la séquence complète, et ainsi de suite, la probabilité de présence d'un élément $B_j$ en un emplacement i dans la séquence complète étant égale à :

$$P_p^i(Bj) = \frac{P_{p-1}^i(Bj) \cdot P(S_p/Bj)}{\displaystyle\sum_{j=1}^{4} P_{p-1}^i(Bj) \cdot P(Sp/Bj)} \quad ,$$

$P_p^i(Bj)$ étant la probabilité de la présence de l'élément Bj à l'emplacement i de la séquence complète après considération de p séquences lacunaires,

$$P_{p-1}^i(Bj)$$

étant la probabilité de la présence de l'élément Bj à l'emplacement i de la séquence complète après considération de (p-1) séquences lacunaires,

$P(S_p/Bj)$ étant la probabilité de la séquence lacunaire $S_p$, connaissant l'élément Bj à l'emplacement i dans la séquence complète.

2. Procédé de séquençage selon la revendication 1, caractérisé en ce qu'après exécution des calculs de probabilité correspondant à un certain nombre de séquences lacunaires, il consiste à poursuivre ces calculs en reprenant celles de ces séquences lacunaires qui présentent le plus grand nombre d'éléments marqués.

3. Procédé de séquençage selon la revendication 1 ou la revendication 2, caractérisé en ce qu'il consiste, préalablement, à classer les séquences lacunaires par nombre décroissant d'éléments marqués et à commencer les calculs de probabilité en considérant d'abord les séquences lacunaires qui présentent le plus grand nombre d'éléments marqués.

4. Procédé de séquençage selon l'une quelconque des revendications 1 à 3, caractérisé en ce qu'il consiste à déterminer préalablement le nombre N d'éléments de la séquence complète.

5. Procédé de séquençage selon l'une quelconque des revendications 1 à 4, caractérisé en ce qu'il consiste à déterminer préalablement, pour chaque type d'élément, le nombre total d'éléments de ce type dans la séquence complète.

6. Procédé de séquençage selon la revendication 5, caractérisé en ce que les nombres d'éléments de chaque type dans la séquence complète sont déterminés par voie chimique.

7. Procédé de séquençage selon la revendication 5 ou la revendication 6, caractérisé en ce qu'on initialise le calcul des probabilités à partir des nombres d'éléments de chaque type dans la séquence complète, la valeur initiale de la probabilité de la présence d'un élément Bj en un emplacement donné étant égale à nj/N, nj étant le nombre total d'éléments Bj dans la séquence complète et N le nombre total d'éléments dans cette séquence.

8. Procédé de séquençage selon l'une quelconque des revendications 1 à 7, caractérisé en ce que N est compris entre 10 et 30 environ.

9. Procédé de séquençage selon l'une quelconque des revendications 1 à 8, caractérisé en ce qu'il consiste à considérer dans une séquence biologique des séquences complètes de N éléments qui se recouvrent mutuellement à leurs extrémités, et à déterminer de la façon précitée les suites d'éléments constituant ces séquences successives, N étant compris entre 10 et 30 environ.

**Claims**

1. Method for sequencing the elements constituting a linear or linearised and ordered biological sequence such as, for example, a DNA fragment, this method consisting, in particular, in extracting, purifying and, where appropriate, fragmenting and/or amplifying the said biological sequence in order to obtain a plurality of identical sequences, in combining suitable specific labels with the elements constituting these sequences, and then in identifying sequentially and counting the labels combined with the said elements in order to deduce therefrom a complete sequence of elements, characterised in that it consists in:

- labelling the said sequences with the set of specific labels mentioned above, the efficiency of this labelling being less than 100%,
- detecting and identifying sequentially the labels combined with the elements of the said sequences, so as to obtain lacunar or incomplete sequences of labelled elements, which sequences differ from one another in respect of the labelled elements, each of which sequences comprises a number of labelled elements lower than the number N of elements constituting the said biological sequence, and in each of which sequences the spaces between labelled elements have unknown lengths, and
- reconstituting the ordered succession of elements constituting the complete sequence, by calculating from the said lacunar sequences the probabilities of presence of the elements in the complete sequence, by calculating the probabilities of presence of the elements in the complete sequence, starting from predetermined initial values of these probabilities and taking into account the labelled elements present in a first lacunar sequence, and then in modifying the probabilities thereby obtained by taking into account the labelled elements present in a second lacunar sequence, and so on, until probabilities equal to 1 or close to 1 are arrived at for each element of the complete sequence, the abovementioned calculations being performed proceeding element by element and position by position of the complete sequence, calculating the probability of presence of each possible element for a given position, and then the probability of presence of each of the possible elements for the next position in the complete sequence, and so on, the probability of presence of an element Bj at a position i in the complete sequence being equal to:

$$P_p^i\,(Bj) \;=\; \frac{P_{p-1}^i(Bj)\,.\,P(S_p^i/Bj)}{\displaystyle\sum_{j=1}^{4}\,P_{p-1}^i(Bj)\,.\,P(S_p/Bj)}\;,$$

$P_p^i\,(Bj)$ being the probability of the presence of the element Bj at position i of the complete sequence after consideration of p lacunar sequences,

$$P_{p-1}^i\,(Bj)$$

being the probability of the presence of the element Bj at position i of the complete sequence after consideration of (p-1) lacunar sequences,

$P(S_p/Bj)$ being the probability of the lacunar sequence $S_p$, knowing the element Bj at position i in the complete sequence.

2. Sequencing method according to Claim 1, characterised in that, after carrying out the calculations of probability corresponding to a certain number of lacunar sequences, it consists in continuing these calculations by dealing with those lacunar sequences which possess the largest number of labelled elements.

3. Sequencing method according to Claim 1 or to Claim 2, characterised in that it consists in classifying the lacunar sequences beforehand on the basis of decreasing number of labelled elements, and in beginning the calculations of probability by considering first the lacunar sequences which possess the largest number of labelled elements.

4. Sequencing method according to any one of Claims 1 to 3, characterised in that it consists in determining beforehand the number N of elements of the complete sequence.

5. Sequencing method according to any one of Claims 1 to 4, characterised in that it consists in determining beforehand, for each type of element, the total number of elements of this type in the complete sequence.

6. Sequencing method according to Claim 5, characterised in that the numbers of elements of each type in the complete sequence are determined chemically.

7. Sequencing method according to Claim 5 or Claim 6, characterised in that the calculation of the probabilities is begun from the numbers of elements of each type in the complete sequence, the initial value of the probability of the presence of an element Bj at a given position being equal to nj/N, nj being the total number of elements Bj in the complete sequence and N the total number of elements in this sequence.

8. Sequencing method according to any one of Claims 1 to 7, characterised in that N is between 10 and 30 approximately.

9. Sequencing method according to any one of Claims 1 to 8, characterised in that it consists in considering, in a biological sequence, complete sequences of N elements which mutually overlap at their ends, and in determining in the abovementioned manner the successions of elements constituting these successive sequences, N being between 10 and 30 approximately.

**Patentansprüche**

1. Verfahren zur Sequenzierung der Bestandteile, die eine lineare oder linearisierte und geordnete biologische Sequenz bilden, wie beispielsweise ein DNA-Fragment, wobei man insbesondere die biologische Sequenz extrahiert, reinigt und gegebenenfalls spaltet und/oder vervielfältigt, um eine Vielzahl von identischen Sequenzen zu erhalten, die diese Sequenzen bildenden Bestandteile mit geeigneten spezifischen Markern versieht, anschließend sequentiell identifiziert und die mit den Markern assoziierten Bestandteile zählt, um daraus eine vollständige Sequenz der Bestandteile abzuleiten, dadurch **gekennzeichnet,** daß man
   - die Sequenzen durch die Gesamtheit der vorstehend genannten spezifischen Marker markiert, wobei die Effizienz dieser Markierung unter 100% liegt,
   - stufenweise die mit den Bestandteilen der Sequenzen verbundenen Marker nachweist und identifiziert, um kettenförmige oder unvollständige Sequenzen der markierten Bestandteile zu erhalten, die sich voneinander durch die markierten Bestandteile unterscheiden, die jeweils eine Anzahl von markierten Bestandteilen unter der Anzahl N der Bestandteile, die die biologische Sequenz bilden, umfassen, und worin in jedem davon die Intervalle zwischen den markierten Bestandteilen unbekannte Längen besitzen, und
   - man die geordnete Folge der Bestandteile, die die vollständige Sequenz bilden, aufstellt, indem man ausgehend von den kettenförmigen Sequenzen die Wahrscheinlichkeiten des Vorhandenseins von Bestandteilen in der vollständigen Sequenz berechnet, indem man von vorbestimmten Anfangswerten dieser Wahrscheinlichkeiten ausgeht und die in einer ersten kettenförmigen Sequenz vorhandenen markierten Bestandteile berücksichtigt, anschließend die so erhaltenen Wahrscheinlichkeiten modifiziert, indem man die in einer zweiten kettenförmigen Sequenz vorhandenen markierten Bestandteile berücksichtigt usw. bis man zu Wahrscheinlichkeiten, die gleich 1 oder nahe 1 für jeden Bestandteil der vollständigen Sequenz sind, gelangt, wobei die vorstehenden Berechnungen dadurch durchgeführt werden, daß man Bestandteil für Bestandteil und Position für Position der vollständigen Sequenz vorgeht, indem man die Wahrscheinlichkeit des Vorhandenseins jedes möglichen Bestandteils für eine gegebene Position berechnet, anschließend die Wahrscheinlichkeit des Vorhandenseins jedes der möglichen Bestandteile für die Position entsprechend der vollständigen Sequenz berechnet usw., wobei die Wahrscheinlichkeit des Vorhandenseins eines Bestandteils Bj an einer Position i in der vollständigen Sequenz gleich

$$P_p^i (Bj) = \frac{P_{p-1}^i(Bj) \cdot P(S_p^i/Bj)}{\sum\limits_{j=1}^{4} P_{p-1}^i(Bj) \cdot P(Sp/Bj)} \quad ,$$

ist,

wobei $P_p^i$ (Bj) die Wahrscheinlichkeit des Vorhandenseins des Bestandteils Bj an der Position i der vollständigen Sequenz nach Berücksichtigung von p kettenförmigen Sequenzen ist,

$$P_{p-1}^i (Bj)$$

die Wahrscheinlichkeit des Vorhandenseins des Bestandteils Bj an der Position i der vollständigen Sequenz nach Berücksichtigung von (p-1) kettenförmigen Sequenzen ist,

$P(S_p/Bj)$ die Wahrscheinlichkeit der kettenförmigen Sequenz $S_p$ in Kenntnis des Bestandteils Bj an der Position i in der vollständigen Sequenz ist.

2. Verfahren zur Sequenzierung nach Anspruch 1, dadurch **gekennzeichnet,** daß nach Durchführung der Wahrscheinlichkeitsberechnungen entsprechend einer bestimmten Anzahl kettenförmiger Sequenzen man die Berechnungen fortsetzt, indem man diejenigen der kettenförmigen Sequenzen, die die größte Anzahl markierter Bestandteile besitzen, berücksichtigt.

3. Verfahren zur Sequenzierung nach Anspruch 1 oder 2, dadurch **gekennzeichnet,** daß man zuvor die kettenförmigen Sequenzen durch eine abnehmende Anzahl von markierten Bestandteilen klassifiziert und die Wahrscheinlichkeitsberechnungen beginnt, indem man zuerst die kettenförmigen Sequenzen berücksichtigt, die die größte Anzahl von markierten Bestandteilen besitzen.

4. Verfahren zur Sequenzierung nach einem der Ansprüche 1 bis 3, dadurch **gekennzeichnet,** daß man zuvor die Anzahl N der Bestandteile der vollständigen Sequenz bestimmt.

5. Verfahren zur Sequenzierung nach einem der Ansprüche 1 bis 4, dadurch **gekennzeichnet,** daß man zuvor für jeden Typ von Bestandteil die Gesamtanzahl der Bestandteile dieses Typs in der vollständigen Sequenz bestimmt.

6. Verfahren zur Sequenzierung nach Anspruch 5, dadurch **gekennzeichnet,** daß die Anzahlen der Bestandteile jedes Typs in der vollständigen Sequenz chemisch bestimmt werden.

7. Verfahren zur Sequenzierung nach Anspruch 5 oder 6, dadurch **gekennzeichnet,** daß man die Wahrscheinlichkeitsberechnung ausgehend von den Anzahlen der Bestandteile jedes Typs in der vollständigen Sequenz beginnt, wobei der anfängliche Wahrscheinlichkeitswert des Vorhandenseins eines Bestandteils Bj an einem gegebenen Ort gleich nj/N ist, wobei nj die Gesamtzahl der Bestandteile Bj in der vollständigen Sequenz ist, und N die Gesamtzahl der Bestandteile in dieser Sequenz ist.

8. Verfahren zur Sequenzierung nach einem der Ansprüche 1 bis 7, dadurch **gekennzeichnet,** daß N zwischen etwa 10 und 30 liegt.

9. Verfahren zur Sequenzierung nach einem der Ansprüche 1 bis 8, dadurch **gekennzeichnet,** daß man in einer biologischen Sequenz die vollständigen Sequenzen aus N Elementen berücksichtigt, die sich wechselseitig an ihren Enden bedecken und wie vorstehend beschrieben die Folgen der Bestandteile, die diese aufeinanderfolgenden Sequenzen bilden, bestimmt, wobei N zwischen etwa 10 und 30 liegt.

```
                    ┌─────────────────────┐
                    │  Extraction         │   ╱ 10
                    │  purification       │
                    │  amplification      │
                    └─────────────────────┘
                              ↓
                    ┌─────────────────────┐   ╱ 12
                    │     marquage        │
                    └─────────────────────┘
                              ↓
                    ┌─────────────────────┐   ╱ 14
                    │  détection/lecture  │
                    └─────────────────────┘
                              ↓
                    ┌─────────────────────┐
                    │  séquences lacunaires│
                    │  S1 = tgagccat      │
                    │  S2 = ccattgactg    │
                    │  S3 = gatg          │
                    │    .                │   ╱ 16
                    │    .                │
                    │    .                │
                    │  Sp = gttgact       │
                    │    :                │
                    └─────────────────────┘
                              ↓
                    ┌─────────────────────┐
                    │  nA, nC, nG, nT     │   ╱ 18
                    │  N = nA + nC + nG + nT│
                    └─────────────────────┘
                              ↓
```

Calcul des probabilités à partir de $n_A$, $n_C$, $n_G$, $n_T$ et de S1

$$P_1^1(A) \quad P_1^2(A) \quad \ldots\ldots \quad P_1^N(A)$$

$$P_1^1(C) \quad P_1^2(C) \quad \ldots\ldots \quad P_1^N(C)$$

$$P_1^1(G) \quad P_1^2(G) \quad \ldots\ldots \quad P_1^N(G)$$

$$P_1^1(T) \quad P_1^2(T) \quad \ldots\ldots \quad P_1^N(T)$$

╱ 20

# FIG.1A

↓

Calcul des probabilités à partir des

$P_1^i(Bj)$ et de S2

$P_2^1(A)$      $P_2^2(A)$ ....... $P_2^N(A)$

$P_2^1(C)$      ............ $P_2^N(C)$

$P_2^1(G)$      ............ $P_2^N(G)$

$P_2^1(T)$      ............ $P_2^N(T)$

↓

itération du calcul jusqu'à obtention
d'une probabilité égale à 1 pour
chaque emplacement d'une base

```
A = 1 0 0 0 0 --------
C = 0 0 1 1 0 --------
G = 0 0 0 0 1 --------
T = 0 1 0 0 0 --------
```

↓

obtention de la séquence complète
ATCCGCATTT-----

# FIG. 1B